# EUROPEAN PATENT SPECIFICATION

(11) **EP 3 949 980 B1**
(45) Date of publication and mention of the grant of the patent: **29.10.2025**
(21) Application number: 21189331.8
(22) Date of filing: 03.08.2021
(51) Int. Cl.: A61K 38/44, A61K 8/92, A61K 47/44, A61P 17/00, A61Q 19/00, A61K 9/06, A61K 9/10, A61K 9/14, A61K 9/70, A61K 9/107

(54) **COMPOSITIONS FOR THE TREATMENT OF ATOPIC DERMATITIS CONTAINING OZONATED OILS AND NATURAL ANTIOXIDANT AGENTS**
ZUSAMMENSETZUNGEN ZUR BEHANDLUNG VON ATOPISCHER DERMATITIS, DIE OZONISIERTE ÖLE UND NATÜRLICHE ANTIOXIDANTIEN ENTHALTEN
COMPOSITIONS POUR LE TRAITEMENT DE LA DERMATITE ATOPIQUE CONTENANT DES HUILES OZONISÉES ET DES AGENTS ANTIOXYDANTS NATURELS

(30) Priority: 04.08.2020 IT 202000019228
(43) Date of publication of application: 09.02.2022
(73) Proprietor: Griva, Vassiliki, 27100 Pavia (IT)
(72) Inventor: Griva, Vassiliki, 27100 Pavia (IT)
(74) Representative: Botti & Ferrari S.p.A.

(56) References cited:
- EP-A1- 1 273 295
- WO-A1-2012/120454
- WO-A2-2005/121328
- JP-A- 2018 158 946
- US-A1- 2014 335 155
- LU J. ET AL: "A preliminary study on topical ozonated oil in the therapeutic management of atopic dermatitis in murine", vol. 29, no. 7, 3 October 2018 (2018-10-03), UK, pages 676 - 681, XP055791133, ISSN: 0954-6634, Retrieved from the Internet <URL:http://dx.doi.org/10.1080/09546634.2018.1443199> [retrieved on 20210329], DOI: 10.1080/09546634.2018.1443199

## Description

The present invention relates to compositions for the treatment of atopic dermatitis containing ozonated oils and natural antioxidant agents, in particular thioredoxin-rich extracts of fermented sake.

### Background of the invention

Atopic dermatitis (AD) is a chronic relapsing inflammatory skin disease, characterized by dryness (xerosis) and skin hyper-reactivity with intense itching. It occurs in 70-80% of cases in the first year of life, often around the 3rd-4th month, but it can also begin in adulthood; In 30-50% of children it heals spontaneously at 3-4 years but can persist afterwards. The etiopathogenesis is multifactorial, with a complex interaction of genetic and environmental factors.

Regarding genetic factors, the family history is observed to be positive for atopic diseases in 70-80% of patients and for AD in 30% of cases.

Environmental factors include immunological factors, represented by food allergens (food allergy is the predominant cause in the first year of life, more rarely in the following years), inhalant allergens, microbial antigens; non-immunological factors, due to exposure of the skin to irritants and to psychological stress conditions and in situations involving an increase in body temperature (physical activities, sweating).

Two forms of AD have been identified: an extrinsic form (60-80% of patients), which is associated with the presence of IgE that are specific towards food or inhalant allergens and with an evolution trend towards other atopic diseases (asthma, oculorinitis, allergic conjunctivitis and allergic gastroenteritis), and an intrinsic form (20-40% of patients), which is not associated with the presence of specific IgE.

AD therapy is principally topical. The drugs used for this purpose are mainly emollients, corticosteroids, immunomodulators, antibiotics and non-steroidal anti-inflammatory drugs.

Emollients should be applied regularly, at least twice a day, in each stage of the disease, in order to moisturize the dry skin of the atopic patient and to promote the re-establishment of the epidermal barrier function. They are contained in a wide range of commercially available products, possibly associated with other ingredients.

Topical dermatological corticosteroids are the most effective therapeutic means in the inflammatory stages of medium-severe AD. In particular, mometasone furoate and methylprednisolone aceponate have the advantage of being administered once a day, with reduced side effects due to reduced systemic absorption. However, in topical corticosteroid treatments, applications should be avoided on the face at any age, on the groin areas and on the diaper area in infants; in the adolescent the applications should be avoided on the inner regions of the thighs, due to the risk of stretch marks.

Topical non-steroidal anti-inflammatories can be used to overcome symptomatic phases of AD without resorting to corticosteroid drugs. For example, furalglucitol shows an anti-inflammatory action by activating free radicals.

Topical immunomodulators such as tacrolimus and pimecrolimus have been employed in children over 2 years of age.

In case of local impetigo manifestations, the confined application of topical antibiotics, such as mupirocin and fusidic acid, can be used.

In addition to topical therapy, systemic therapy with antihistamines, systemic corticosteroids and oral antibiotics can also be used.

Recently, compositions useful for the treatment of allergic or radiation dermatitis containing as active ingredient thioredoxin, an antioxidant protein present in eukaryotic cells (for example, reference can be made to patent applications JP 2011001269, JP2007069671, KR 20100046605, WO 2005/121328, US 2014/335155, EP 1758990) have also been described.

The use of ozonated oils for the treatment of several conditions is also known, including some conditions of dermatological interest, especially in the cosmetic field. For example, reference can be made to patent applications KR 20180013659, EP 1273295, EP 2683356, CN 108653320). Soaps containing ozonated oils have been described in JP 2009091489 and in CN 107714573.

In any case, a treatment of atopic dermatitis, that exploits safe and effective active ingredients that allow a long-term treatment without the typical side effects of corticosteroids, immunosuppressants and other drugs used nowadays, remains desirable.

### Description of the invention

It has now been found that the association of ozonated oils with extracts containing thioredoxin is particularly effective in the topical treatment of atopic dermatitis, in particular in pediatric patients.

The present invention therefore relates to topical compositions containing effective amounts of ozonated oils and extracts comprising thioredoxin in a mixture with any additional ingredients and with conventional excipients.

Ozonated oils are known and commercially available. An oil ozonation process is described in WO 2012168770.

Examples of ozonated oils are sunflower oil, extra virgin olive oil, wheat germ oil, hemp oil, jojoba oil.

A preferred oil is sunflower oil, available from the producer Rapetti.

A preferred thioredoxin source is the fermented sakè extract commercially available under the designation of Thioredoxyl^{®}, the preparation of which is described in US 8450099.

In addition to the ozonated oils and the extract containing thioredoxin, other active ingredients with complementary, synergistic or otherwise useful activity could be present, such as anti-inflammatory, soothing and moisturizing agents.

Examples of said other ingredients include wheat germ oil extracts, ceramides, panthenol, collagen, chamomile oil, alpha bisabolol, hyaluronic acid, urea, 18-beta glycyrrhetinic acid, zantalene, zinc oxide, argan oil, shea butter, beta sitosterol.

The compositions of the invention can be formulated in creams, ointments, gels, lotions, sprays, powders and other forms suitable for topical administration on injured skin, including gauze, tissues or patches on which the composition of the invention is adsorbed.

The concentration of ozonated oil can range from 0.1 to 30% by weight, preferably from 1 to 30%, more preferably from 3 to 25%.

The concentration of fermented sake extract can range from 0.1 to 0.5% by weight, preferably from 0.1 to 0.5%, more preferably 0.2% by weight.

The following examples describe the invention in more detail.

### Example 1 - emulsion in oil

- ozonated *sunflower oil 26%*
- thioredoxin (*Bio-Redoxyl* ^{®}) 0.2%
- argan oil 6%
- wheat germ oil 3%
- shea butter 4%
- beta sitosterol 2%
- ceramides 2%
- panthenol 1%
- marine collagen 0.3%
- chamomile e.g. 1%
- alpha bisabolol 0.1%
- hyaluronic acid sodium salt 0.2%
- urea 5%
- 18-beta glycyrrhetinic acid 0.2%
- zantalene 0.3%
- zinc oxide 5%
- sunflower oil up to 100%

### Example 2 - Clinical trials

The synergistic effect of the association of thioredoxin (0.2% by weight) and ozonated sunflower oil (25% by weight) has been confirmed in clinical trials on children and adults.

### Pediatric clinical cases

Both infants between six and eight months of age (infants) and children between two and eight years were analyzed.
- An eight-month-old female child suffered from severe diaper dermatitis for four months, resistant to various applications of corticosteroid therapy, on the entire perianal area, on the buttocks, in the groin and on the external genitals.

1st treatment: after application of ozonated oil twice a day some signs of improvement were noticed after three days.

2nd treatment: after application of thioredoxin twice a day for three days a greater improvement compared to the treatment with ozonated oil is found.

3rd treatment: after application of the association of ozonated sunflower oil and thioredoxin twice a day, a considerable improvement in symptoms was noted, already on the third day. After increasing the applications to three times / day for another four days, the child was completely healed. After the applications, the perianal and inguinal area was also clearly healed.
- Three-year-old female child with atopic dermatitis on the face and on the upper limbs (right and left forearm).

1st treatment: after application of ozonated oil twice a day, signs of improvement were noticed on the third day.

2nd treatment: after application of thioredoxin twice a day for three days a greater improvement compared to the treatment with ozonated oil is found.

3rd treatment: the application of ozonated sunflower oil and thioredoxin twice a day for five days resulted in an improved clinical picture after only three days.
- Five-year-old male child with eczematous atopic dermatitis on the upper limbs, elbows, bilateral forearms, associated with intense itching and sometimes skin rash on the face and legs.

1st treatment: after application of ozonated oil twice a day signs of improvement were noticed after four days.

2nd treatment: after application of thioredoxin twice a day for three days a greater improvement compared to the treatment with ozonated oil was found.

3rd treatment: the application of ozonated sunflower oil and thioredoxin twice a day for seven days resulted in a significant improvement in symptoms.
- Six-month-old infant with eczematoid atopic dermatitis on the face, trunk, upper and lower limbs.

1st treatment: after application of ozonated oil twice a day, improvements were noted after three days.

2nd treatment: after application of thioredoxin twice a day for three days a greater improvement compared to the treatment with ozonated oil was found.

3rd treatment: the application of ozonated sunflower oil and thioredoxin three times a day, on the third day a clear regression of the clinical symptoms was noted. After five days the symptoms disappeared.
- Seven-year-old female child with bilateral atopic dermatitis on the legs and upper limbs (forearms and arms).

1st treatment: after application of ozonated oil twice a day some signs of improvement were noticed after three days.

2nd treatment: after application of thioredoxin twice a day for three days a greater improvement compared to the treatment with ozonated oil was found.

3rd treatment: the application of ozonated sunflower oil and thioredoxin three times a day resulted in a marked improvement in symptoms after only five days.

### Clinical cases in adult patients

Seven patients with eczematous atopic dermatitis were treated; five of these patients had moderate bilateral atopic dermatitis in the upper (forearms, arms) and lower (legs and thighs) limbs and two of these patients had dyshidrosis and severe atopic dermatitis in the hands and feet.

Also in these cases, after treatment with the association of ozonated sunflower oil and thioredoxin three times a day for seven days, a marked improvement was highlighted in the clinical picture with recovery of the underlying skin from lichenization and de-epithelialization.

### Conclusions

The results show that the association of thioredoxin (0.2%) with ozonated sunflower oil (25%) has a higher clinical efficacy than thioredoxin and the ozonated oil individually.

## Claims

1. Topical compositions containing ozonated oils in association with extracts comprising thioredoxin mixed with any other ingredients and with conventional excipients.

2. Compositions according to claim 1 wherein the extract containing thioredoxin is a fermented sake extract.

3. Compositions according to claims 1 or 2 wherein the ozonated oil is sunflower oil, extra virgin olive oil, wheat germ oil, hemp oil, jojoba oil.

4. Compositions according to one or more of claims 1 to 3 comprising one or more components selected from extracts of wheat germ oil, ceramides, panthenol, collagen, chamomile oil, alpha-bisabolol, hyaluronic acid, urea, 18-beta glycyrrhetinic acid, zantalene, zinc oxide, argan oil, shea butter, beta sitosterol.

5. Compositions according to one or more of claims 1 to 4 in the form of gel, cream, ointment, powder, lotion, gauze, patch or fabric.

6. Compositions according to one or more of claims 1 to 5 wherein the concentration of ozonated oil ranges from 0.1 to 30% by weight.

7. Compositions according to one or more of claims to 6 wherein the concentration of fermented sake extract ranges from 0.1 to 0.5% by weight.

8. Compositions of claims 1-7 for use in the treatment of atopic dermatitis.

## Patentansprüche

1. Topische Zusammensetzungen, die ozonisierte Öle in Verbindung mit Extrakten enthalten, die Thioredoxin in Mischung mit anderen Inhaltsstoffen und herkömmlichen Hilfsstoffen umfassen.

2. Zusammensetzungen gemäß Anspruch 1, wobei der Thioredoxin enthaltende Extrakt ein fermentierter Sake-Extrakt ist.

3. Zusammensetzungen gemäß Anspruch 1 oder 2, wobei das ozonisierte Öl Sonnenblumenöl, natives Olivenöl extra, Weizenkeimöl, Hanföl oder Jojobaöl ist.

4. Zusammensetzungen gemäß einem oder mehreren der Ansprüche 1 bis 3, die einen oder mehrere Bestandteile enthalten, die aus Extrakten von Weizenkeimöl, Ceramiden, Panthenol, Kollagen, Kamillenöl, Alpha-Bisabolol, Hyaluronsäure, Harnstoff, 18-beta-Glycyrrhetinsäure, Zantalene, Zinkoxid, Arganöl, Sheabutter und Beta-Sitosterol ausgewählt sind.

5. Zusammensetzungen gemäß einem oder mehreren der Ansprüche 1 bis 4 in Form von Gel, Creme, Salbe, Pulver, Lotion, Gaze, Pflaster oder Gewebe.

6. Zusammensetzungen gemäß einem oder mehreren der Ansprüche 1 bis 5, wobei die Konzentration des ozonisierten Öls im Bereich von 0,1 bis 30 Gew.-% liegt.

7. Zusammensetzungen gemäß einem oder mehreren der Ansprüche 2 bis 6, wobei die Konzentration des fermentierten Sake-Extrakts zwischen 0,1 und 0,5 Gew.-% liegt.

8. Zusammensetzungen gemäß den Ansprüchen 1 bis 7 zur Verwendung bei der Behandlung von atopischer Dermatitis.

## Revendications

1. Compositions topiques contenant des huiles ozonées en association avec des extraits comprenant de la thiorédoxine mélangée à d'autres ingrédients et à des excipients classiques.

2. Compositions selon la revendication 1, dans lesquelles l'extrait contenant de la thiorédoxine est un extrait de saké fermenté.

3. Compositions selon les revendications 1 ou 2, dans lesquelles l'huile ozonée est de l'huile de tournesol, de l'huile d'olive extra vierge, de l'huile de germe de blé, de l'huile de chanvre ou de l'huile de jojoba.

4. Compositions selon une ou plusieurs des revendications 1 à 3, comprenant un ou plusieurs composants choisis parmi les extraits d'huile de germe de blé, les céramides, le panthénol, le collagène, l'huile de camomille, l'alpha-bisabolol, l'acide hyaluronique, l'urée, l'acide 18-bêta-glycyrrhétinique, le zantalène, l'oxyde de zinc, l'huile d'argan, le beurre de karité, le bêta-sitostérol.

5. Compositions selon une ou plusieurs des revendications 1 à 4, sous forme de gel, crème, pommade, poudre, lotion, gaze, patch ou tissu.

6. Compositions selon une ou plusieurs des revendications 1 à 5, dans lesquelles la concentration en huile ozonée est comprise entre 0,1 et 30 % en poids.

7. Compositions selon une ou plusieurs des revendications 2 à 6, dans lesquelles la concentration en extrait de saké fermenté est comprise entre 0,1 et 0,5 % en poids.

8. Compositions des revendications 1 à 7, pour une utilisation dans le traitement de la dermatite atopique.
